# EUROPÄISCHE PATENTSCHRIFT

(11) **EP 1 125 566 B1**
(45) Veröffentlichungstag und Bekanntmachung des Hinweises auf die Patenterteilung: **10.11.2004**
(21) Anmeldenummer: 01103795.9
(22) Anmeldetag: 16.02.2001
(51) Int. Cl.: A61F 5/00, A61F 5/03

(54) **Schlankheitsgurt**
Slimming belt
Ceinture amincissante

(30) Priorität: 17.02.2000 DE 20002875 U
(43) Veröffentlichungstag der Anmeldung: 22.08.2001
(73) Patentinhaber: Gomoluch, Jan M., 88299 Leutkirch (DE)
(72) Erfinder: Gomoluch, Jan M., 88299 Leutkirch (DE)
(74) Vertreter: Pfister, Helmut, Dipl.-Ing.

(56) Entgegenhaltungen:
- GB-A- 2 152 383
- US-A- 2 449 641
- US-A- 4 849 863
- US-A- 5 518 009

## Beschreibung

Die Erfindung betrifft einen Schlankheitsgurt, bestehend aus einem vorderen Teil und einem hinteren Teil, wobei der vordere Teil im Bauchbereich angeordnet wird.

Aus ästhetischen oder kosmetischen Gründen ist es häufig wünschenswert, die Figur insbesondere im Bauchbereich zu korrigieren. Zu diesem Zwecke sind verschiedene Vorrichtungen in Gürtel- oder ähnlicher -form bekannt geworden, die auf unterschiedliche weise versuchen, einen Schlankheits- oder Unterstützungseffekt zu erzielen.

In der GB-PS 21 52 383 ist eine Vorrichtung mit Methode zum Gewichtsverlust beschrieben, bei der eine Art Polster verwendet wird, das durch Druck auf Bauch oder Magen ein gewisses Völlegefühl hervorruft und so appetitzügelnd wirkt. Dieses Polster wird mit Hilfe eines Gürtels an der Taille befestigt, wobei dieser, um den Druck auf den Magen auszuüben, entsprechend. stramm gezogen werden muß. Das macht das Tragen unangenehm, insbesondere ist ein solches Polster auf dem Bauch sichtbar, auch wenn es unter der Kleidung getragen wird und erzielt optisch genau den gegenteiligen Effekt von dem, der gewünscht wird. Die Figur erscheint nicht schlanker, sondern insbesondere im Bauchbereich voluminöser. Da die Wirkung dieser Vorrichtung nur so lange anhält, so lange der Gurt getragen wird, wird der Benutzer, sobald er den Gürtel mit dem Polster abgelegt hat, dazu verführt, Essen im Übermaß zu sich zu nehmen, da er nun endlich ein Befreiungsgefühl empfindet. Zudem ist die ganze Vorrichtung kompliziert zum Anlegen, insbesondere da bei einer Ausführungsvariante das Polster aufblasbar ist.

In der US-PS 24 49 641 wird eine Unterstützung für den Bauchbereich vorgeschlagen, die speziell für Patienten mit Leistenbrüchen vorgesehen ist, um entweder einen Leistenbruch zu unterstützen oder diesem vorzubeugen. Auch dieser Gurt ist aus mehreren Einzelteilen zusammengesetzt, was das Anlegen kompliziert macht, zudem ist das Tragen des Gurtes auf einen medizinischen Zweck gerichtet und er versucht keine Schlankheitswirkung zu erreichen. Durch die Verarbeitung von Metall im Gurt ergeben sich schlechte Trageeigenschaften, so daß diese Vorrichtung nur für medizinische Zwecke in Frage kommt.

Die DE-OS 198 25 693 sieht ein kombiniertes Mittel vor, mit dem sich insbesondere Cellulite an den Beinen bekämpfen lässt. Um den gewünschten Effekt zu erzielen ist es aber notwendig, eine Beinkleidung, beispielsweise Strümpfe oder dergleichen, zusammen mit einem Mieder zu kombinieren, wobei die Druckausübung auf den Körper sowohl von Strümpfen wie vom Mieder die Wirkung unterstützen. Um den gewünschten Effekt zu erzielen, muß eine relativ komplizierte Kleidung, bestehend aus mehreren Teilen getragen werden, die bezüglich der Druckausübung auf den Körper aufeinander abgestimmt werden müssen.

Im DE-GBM 296 18 843 wird eine Bandage vorgestellt, die zur Unterstützung der Wirbelsäule geeignet ist. Zu diesem Zwecke ist die Bandage im Rückenbereich stark verbreitert und besteht beispielsweise aus einem nicht atmungsaktiven Kunststoffmaterial wie Neopren. Um eine gewisse Luftzirkulation in diesem Bereich zu erreichen, wird das Neopren beispielsweise mit Löchern versehen, und die Bandage wird als Ganzes mittels eines Verschlusses, beispielsweise eines Klettverschlusses, der im Bauchbereich angeordnet ist, verschlossen. Diese Bandage dient einem anderen Zweck, nämlich der der Unterstützung der Wirbelsäule.

Aus der US-PS 4 849 863 ist ein Schlankheitsgurt bekannt, bei dem auf einem relativ starren Material eine Beschichtung aus nicht atmungsaktivem Material wie Neopren aufgebracht ist, das dazu dient, die Fettzellen, die unterhalb des Bauchgurtes liegen, zum Schwitzen anzuregen.

Die Erfindung hat es sich zur Aufgabe gemacht, einen Schlankheitsgurt der eingangs beschriebenen Art vorzusehen, der bei angenehmen Trageeigenschaften optisch nicht aufträgt und insbesondere im Bauchbereich eine Wirkung erzielt.

Zur Lösung dieser Aufgabe geht die Erfindung aus von einem Schlankheitsgurt wie oben beschrieben und schlägt vor, daß der vordere Teil des Schlankheitsgurtes aus einem nicht atmungsaktiven Material besteht, wobei der vordere Teil aufgrund der eingeschränkten Luftzirkulation des nicht atmungsaktiven Materials für den Bauchbereich durchblutungsfördernd wirkt und so der Entschlackung dient. Der hintere Teil des Schlankheitsgurtes ist zumindest teilweise atmungsaktiv.

Der Schlankheitseffekt eines solchen Gurtes ergibt sich aus dem Schwitzen des Benutzers im Bauchbereich, ebenso wie der Kreislaufanregung in diesem Bereich, wodurch ein Abnehmeffekt in angenehmer Weise erreicht wird. Da der Gurt in etwa gleichförmig ist und unter der Kleidung nicht aufträgt, kann er jederzeit getragen werden, wodurch sich der Effekt erhöht. Bevorzugt besteht der Gurt aus elastischem, textilähnlichem biegsamem Material, was einen angenehmen Tragekomfort ergibt.

Bei der bevorzugten Ausführungsform der Erfindung ist als Material des vorderen Teiles Neopren oder ein anderes, nicht atmungsaktives Material oder eine Mischung dieser Materalien vorgesehen. Dabei ist das Neopren vorteilhafterweise einseitig und/oder beidseitig, zumindest teilweise mit Stoff beschichtet. Die Verwendung von Stoff erhöht den Tragekomfort und ergibt ein angenehmes Gefühl auf der Haut, gleichzeitig dient der Stoff zum Aufsaugen von Schweiß. Es ist auch möglich das nicht atmungsaktive Material in einen Stoffüberzug einzulegen, was erlaubt, daß der Stoffüberzug gewaschen werden kann. Daneben ist es auch möglich, nicht nur eine Beschichtung, sondern auch eine Belegung oder Kaschierung des Neoprens beziehungsweise des nicht atmungsaktiven Materials vorzusehen.

Es hat sich herausgestellt, daß mit Neopren ein besonders guter, kreislaufstimulierender und zirkluationserhöhender Effekt erreicht werden kann, da das Material relativ fest, aber doch elastisch ist und den erwünschten Schwitzeffekt erreicht. Das Material ist günstig herzustellen und kann problemlos gewaschen werden, wobei die günstigen Trageeigenschaften durch eine Stoffbeschichtung, insbesondere im Inneren des Gurtes, erhöht werden. Bei diesem Stoff handelt es sich beispielsweise um schweißaufsaugendes Material, wie es allgemein bekannt ist. Auch das Äußere des Gurtes kann eine Stoffbeschichtung, zumindest teilweise tragen.

Bei der bevorzugten Ausführungsform der Erfindung besteht das Material des hinteren Teiles, zumindest teilweise, aus elastischem Material und/oder, zumindest teilweise, aus atmungsaktivem Material. Da der Schlankheitseffekt, also vor allem der Schwitzeffekt, in erster Linie im vorderen Teil des Gurtes, nämlich in der Bauchregion erwünscht ist, ist es nicht notwendig, den Gurt rundherum aus Neopren oder dergleichen herzustellen. Um die Trageeigenschaften weiter zu erhöhen, kann im Rückenbereich ein Einsatz vorgesehen sein, der aus elastischem Textilmaterial oder dergleichen besteht. Dieses Material bewirkt eine gewisse Warmhaltung der hinteren Rückenpartie, wodurch die Einsetzbarkeit des Gurtes erhöht wird, ermöglicht aber gleichzeitig eine gewisse Luftzirkulation, so daß der Schwitzeffekt verringert ist. Gleichzeitig wird durch die Verwendung eines atmungsaktiven Materials auf der Rückseite vermieden, daß die Rückenpartie beziehungsweise die Nieren überhitzt werden und dann zum Beispiel empfindlich sind auf Zugluft und dergleichen. Gleichzeitig wird das atmungsaktive Material ebenfalls aus Stoff ausgeführt, was eine entsprechende komfortable Verwendung des Schlankheitsgurtes erlaubt. Die Erfindung bezieht sich dabei sowohl auf einen Gurt, der nur im vorderen Bereich nicht atmungsaktiv ist, wie auch auf einen Gurt, der allseitig aus nicht atmungsaktiven Material besteht.

In gewissen Fällen kann es wünschenswert sein, den Gurt rundum aus nicht atmungsaktivem Material anzufertigen, beispielsweise, wenn der Gurt etwa in einer kleineren Variante am Oberschenkel oder an einer anderen Körperstelle getragen werden soll, aber auch wenn der Gurt, wie oben beschrieben, um die Taille getragen wird.

Bei einer bevorzugten Ausführungsform der Erfindung sind im Bereich des hinteren Teiles Gummistabilisatoren vorgesehen. Hierbei handelt es sich beispielsweise um breite Gummibänder, die am Saum des hinteren Teiles parallel zu diesem festgenäht werden, um ein Verwerfen beziehungsweise Aufrollen des Randes (quer zur Längsrichtung) zu vermeiden.

Bei einer weiteren bevorzugten Ausführungsform der Erfindung ist ein stabilisierender Randsaum oben und/oder unten im Bereich des hinteren Teiles vorgesehen, ebenso oben und/oder unten auch im Bereich des vorderen Teiles. Mit einem solchen Saum wird die gleiche Wirkung erzielt wie mit den Gummistabilisatoren. Der Saum ist noch einfacher anzubringen und es ist gefunden worden, daß die Ausbildung eines Saumes, der gegebenenfalls einen oder mehrere cm stark sein kann, die gleiche stabilisierende Wirkung erbringt, als die Verwendung der vorbeschriebenen Gummistabilisatoren, wobei die Ausgestaltung des Saumes, bei dem textilen, hintenliegenden Teil in einfacher Weise ausgeführt werden kann. Es kann sich auch über den gesamten Randbereich des Gurtes erstrecken.

Es hat sich als besonders günstig erwiesen, wenn der Gurt als Endlosteil ausgebildet ist, also keinerlei Verschlüsse oder dergleichen aufweist. Er wird zum Tragen einfach über den Kopf gestreift oder hosenartig über die Beine nach oben gezogen, wobei der elastische Rückenteil die nötige Weite ergibt. Durch das Fehlen von Verschlüssen und dergleichen ist der Gurt absolut gleichförmig und kann unauffällig unter jeder Kleidung getragen werden.

Bei einer anderen Ausführungsform der Erfindung weist der Gurt am vorderen oder hinteren Teil einen Verschluß, zum Beispiel einen Klettverschluß, auf. Insbesondere bei der Integration des Klettverschlusses (es können ein oder zwei Verschlüsse zum Beispiel seitlich an der Taille vorgesehen werden) in den Übergang zwischen vorderem und hinterem Teil, wird das gleichförmige Äußere des Gurtes kaum beeinflusst, auch das Anbringen an einer anderen Stelle lässt den Gurt insgesamt weiterhin flach erscheinen. Als Verschlüsse kommen neben dem Klettverschluß Druckknöpfe, Knöpfe, Haken, Schnürungen oder dergleichen in Frage. Durch das Anbringen eines Verschlusses wird der Tragekomfort weiter erhöht.

Es hat sich als günstig herausgestellt, wenn der hintere Teil ca. 20% bis 50%, bevorzugt etwa 25% des gesamten Umfanges des Schlankheitsgurtes ausmacht. Dabei kann diese Zahl variieren, je nach gewünschtem Schlankheitseffekt und im Extremfall auch gegen Null gehen. Es hat sich als vorteilhaft herausgebildet, daß der Anteil an nicht atmungsaktivem Material soweit am Körper herumreicht, daß auch die seitlichen Bereiche, also die Taille, von dem Schlankheitsgurt bedeckt sind/ist. Hieraus ergibt sich, daß der Anteil des hinteren Teiles entsprechend geringer ist. Umgekehrt kann dies von Vorteil sein, um so eine übermäßige Überhitzung des Rückenbereiches und der Nieren zu vermeiden, einen größeren Anteil an dem Rückenteil zu machen (ca. bis zu 50%), um einer erhöhten Zugempfindlichkeit im Rückenbereich vorzubeugen. Der Verschluß kann auch in das Rückenteil integriert sein, beispielsweise dieses in zwei Teile teilen, oder es ersetzen.

Bei der bevorzugten Ausführungsform der Erfindung ist zumindest das Material des vorderen Teiles aus textilartigem Material und/oder im Verhältnis zur bedeckten Fläche dünn. Der Gurt weist in der Regel eine Breite von etwa 15 bis 30 cm, bevorzugt ca. 20 bis 25 cm auf, je nach Größe des Benutzers, wobei die Dicke auch im neoprenhaltigen Bereich etwa einen halben bis einen Zentimeter nicht überschreitet. Da der Gurt insbesondere beim Sport getragen werden kann, wobei Durchblutung und Schwitztätigkeit in der Bauchregion besonders angeregt werden und die Entschlackung besonders schnell vor sich geht, ist es günstig für die Bewegungsfreiheit und die Optik, wenn der Gurt ein gleichmäßiges Erscheinungsbild bietet. So kann er wahlweise über oder unter der Kleidung getragen werden. Durch das, zumindest teilweise luftdurchlässige Material am Rücken, entfällt der teilweise unangenehme Effekt des übermäßigen Schwitzens am Rücken und im Nierenbereich, was zu Schmerzen und Erkrankungen in dieser Gegend führen kann. Wird ein Gurt ohne Verschluß verwendet, so besteht auch nicht die Gefahr eines unfreiwilligen Öffnens oder eines Verschleisses des Verschlusses. Durch die Variation des Anteiles an atmungsaktivem Material an dem Rückenteil wird auch erreicht, daß zum Beispiel der Randbereich der Taille mehr oder weniger mit dem nicht atmungsaktiven Material abgedeckt ist und so auch bei der Entschlackung mit berücksichtigt wird. Umgekehrt kann ein größerer Anteil an atmungsaktivem Rückenmaterial günstig für eine nicht so große Überhitzung des Rückenbereiches sein.

Dabei ist es günstig, daß zumindest das Material des vorderen Teiles aus textilartigem, und oder im Verhältnis zur bedeckten Fläche dünnen Material ist. Das im Bereich des Bauches aufliegende Material weist eine gewisse Geschmeidigkeit und Elastizität auf, welche zum einen einen ausreichenden Tragekomfort und auch einen möglichst optisch nicht wahrnehmbaren Einsatz erlaubt.

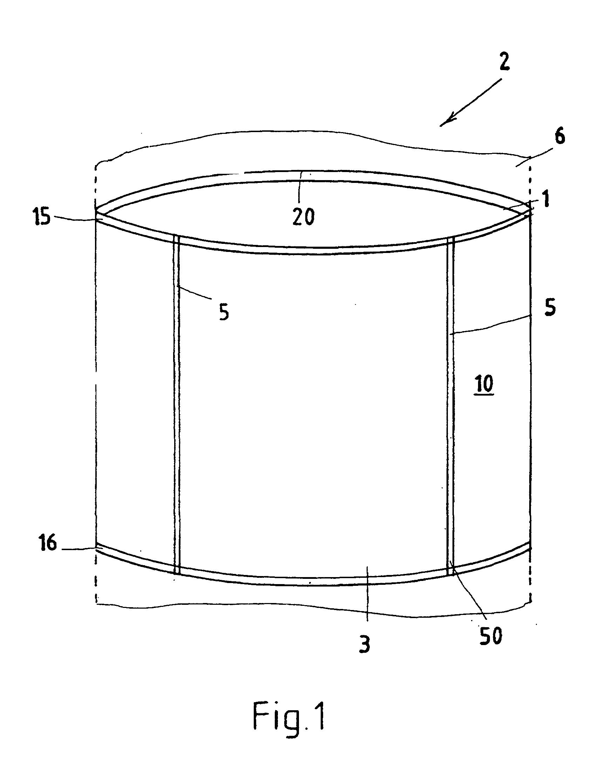

In der Zeichnung ist ein Ausführungsbeispiel des erfindungsgemäßen Schlankheitsgurtes schematisch dargestellt. Es zeigen
- Fig. 1: eine perspektivische Rückansicht eines erfindungsgemäßen Gurtes im Gebrauch,
- Fig. 2: einen Vertikalschnitt durch einen erfindungsgemäßen Gurt gemäß der Erfindung,
- Fig. 3: einen Horizontalschnitt durch einen erfindungsgemäßen Gurt gemäß der Erfindung und
- Fig. 4: eine Draufsicht von hinten auf eine Variation der Erfindung

Der Gurt 2 besteht im wesentlichen aus dem vorderen Teil 1 und dem hinteren Teil 3.

Das Material 10 des vorderen Teiles 1 besteht dabei vorzugsweise aus Neopren oder einem ähnlichen, nicht atmungsaktiven Material, wie Gummi oder einem anderen Kunststoff oder auch aus einem Gemisch solcher Stoffe. Auf der Außenseite ist ein Stoff 11 und der Innenseite ein Stoff 12 als Beschichtung, Kaschierung oder abziebarer Überzug vorgesehen. Bei der Stoffbeschichtung 12 im Inneren des Gurtes 2 handelt es sich vorzugsweise um schweißaufsaugendes Material, wobei Baumwollmaterialien oder auch Kunststoffmaterialien oder Gemische vorgesehen werden können. Auch der Stoff 11 im Außenbereich des Gurtes 2 kann aus schweißaufsaugendem Material bestehen, zusätzlich kann hier ein gewisser optischer Effekt durch den Einsatz gewisser Farben oder Materialien erzielt werden.

Um den vorderen Teil 1 gegen ein Aufrollen des Randes 15, 16 zu schützen, werden Randsäume 13, 14 und unten und oben vorgesehen. Diese Randsäume werden einfach bei der Herstellung des Gurtes 2 angebracht und weisen eine gewisse Breite, beispielsweise etwa einen halben bis einen Zentimeter auf. Nach Belieben können diese auch verstärkt werden. In manchen Fällen ist es nicht notwendig, auf beiden Seiten des Gurtes solche Randsäume anzubringen. Gleichzeitig werden die Randsäume 13, 14 dazu benutzt, das Stoffmaterial 11, 12 mit dem Material 10 des Gurtes zu vernähen und zu versäubern. Auf diese Weise ist die Herstellung besonders einfach. Die Randsäume verhindern ein Aufwerfen des Gurtes an seinem Rand 15, 16, was zu erhöhtem Verschleiß und unschönem Aussehen führt.

Das Material 30 des hinteren Teiles 3 kann beispielsweise aus atmungsaktivem Textilmaterial bestehen, so daß die hintere Rückenpartie des Benutzers, insbesondere beim Sporttreiben, nicht so stark schwitzt und durchblutet wird, wie der Bauchbereich, wo dieser Effekt gewünscht wird. Das Textilmaterial ist zudem dehnbar und erleichtert das Anlegen des Gurtes und besteht entweder selbst aus schweißaufsaugendem, körperangenehmen Material oder kann mit einem solchen Material beschichtet werden.

Durch die Beschaffenheit des Materials 30 im hinteren Teil 3 bedingt kann es notwendig sein, Gummistabilisatoren 31, 32 unten und oben im hinteren Teil 3 anzubringen, um ein Aufwerfen und Einrollen des Randes zu verhindern. Es hat sich herausgestellt, daß in vielen Fällen das Anbringen von Randsäumen 33, 34 ähnlich wie im vorderen Teil 1 ausreichend ist. Mit Hilfe dieser Randsäume 33, 34 wird gleichzeitig das Material 30 versäubert.

Der Umfang 20 des Schlankheitsgurtes 2 entspricht in etwa dem Körperumfang im Bauchbereich des Benutzers, wobei beispielsweise verschiedene Umfanggrößen angeboten werden können. Günstigerweise ist der Umfang 20 des Schlankheitsgurtes 2 etwas geringer gewählt als der Körperumfang im Bauchbereich des Benutzers, dadurch wird ein enges Anliegen des Gurtes am Körper sichergestellt, die Elastizität und Flexibilität des Materials ergibt eine gute Körperanlage und ist unter der Kleidung getragen kaum wahrzunehmen.

Im Bereich der Verbindungsnaht 5 sind der vordere Teil 1 und der hintere Teil 3 miteinander verbunden, beispielsweise fest vernäht. Auch hier können längsverlaufende Stabilisatoren 50 eingesetzt werden, bei denen es sich beispielsweise um breite Textilbänder oder dergleichen handelt, um ein Aufwerfen des Gurtes 2 zu verhindern und gleichzeitig die Stabilität des Gurtes 2 zu erhöhen.

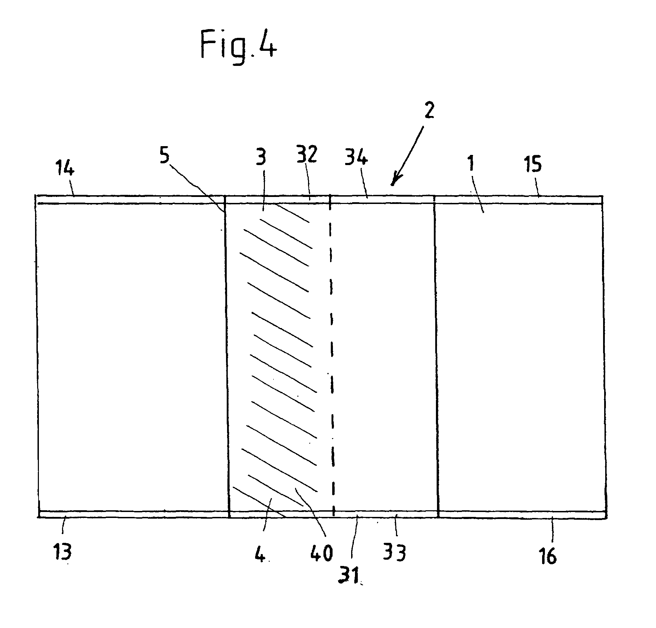

Bei einer anderen Ausführungsvariante der Erfindung ist im Bereich der Verbindungsnaht 5 ein Verschluß 4 vorgesehen, wie in Fig.4 zu sehen ist. Bei diesem Verschluß handelt es sich beispielsweise um einen Klettverschluß 40, wobei das Material 30 in diesem Bereich überlappend ausgebildet ist. Bei dieser Ausführungsform der Erfindung wird der Gurt nicht durch Überstreifen angelegt, sondern kann um die Taille gelegt werden. Als Verbindungsmittel für den Verschluß 4 kommen außer Klettverschluß Knöpfe, Haken, Ösen, Reißverschlüsse, Schnürungen und dergleichen in Frage.

Der Verschluß 4 kann auch im vorderen Teil 1 des Gurtes 2 angebracht sein und dann entweder auf der Bauchseite oder der Flanke des Benutzers zu liegen kommen.

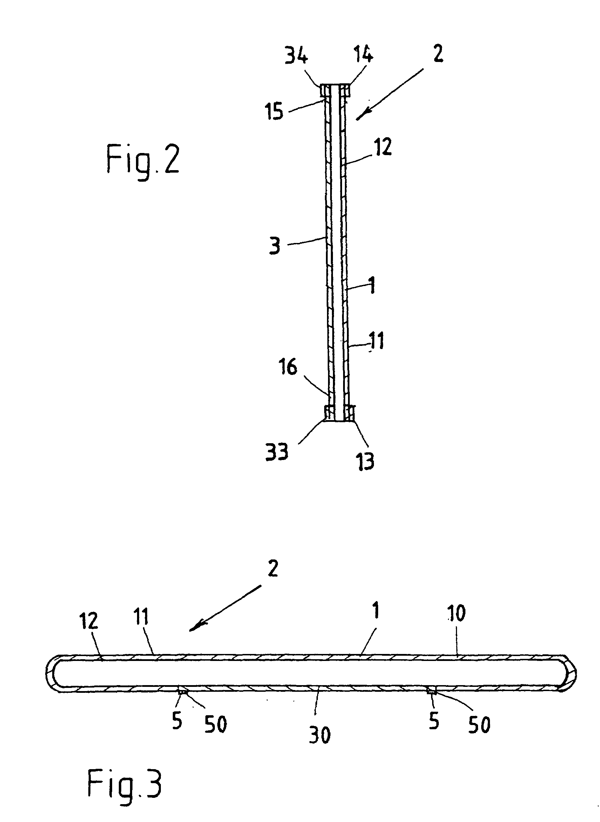

Durch die Auswahl der Materialien 10, 30, die beide mehr oder weniger elastisch sind, passt sich der Schlankheitsgurt 2 gut dem Körper 6 des Benutzers an und ergibt insbesondere im Bauchbereich, wo dies auch erwünscht ist, einen Schwitzeffekt, wodurch Fett und Wasser in dieser Region abgebaut werden. Das Material des Gurtes 2, insbesondere auch das nicht atmungsaktive Material in der Bauchregion, ist vergleichsweise dünn, etwa einen halben Zentimeter, im Vergleich zu der Fläche, die dieses Material bedeckt. Die Breite des Gurtes beträgt etwa 20 bis 25 Zentimeter. Die Ausführung des Gurtes 2 ist im Querschnitt, wie in den Figuren 2 und 3 zu sehen, gleichmäßig, so daß der Gurt 2 auch unter der Kleidung unauffällig getragen werden kann.

## Patentansprüche

1. Schlankheitsgurt, bestehend aus einem vorderen Teil und einem hinteren Teil, wobei der vordere Teil dazu geeignet ist, im Bauchbereich angeordnet zu werden, **dadurch gekennzeichnet, daß** der vordere Teil (1) des Schlankheitsgurtes (2) aus einem nicht atmungsaktiven Material (10) besteht, wobei der vordere Teil aufgrund der eingeschränkten Luftzirkulation des nicht atmungsaktiven Materials (10) für den Bauchbereich durchblutungsfördernd wirkt und so der Entschlackung dient und der hintere Teil (3) zumindest teilweise atmungsaktiv ist.

2. Schlankheitgurt nach Anspruch 1, **dadurch gekennzeichnet, daß** als Material (10) des vorderen Teiles (1) Neopren oder ein anderes nicht atmungsaktives Material oder eine Mischung dieser Materialien vorgesehen ist.

3. Schlankheitsgurt nach einem oder beiden der vorhergehenden Ansprüche, **dadurch gekennzeichnet, daß** das Neopren beziehungsweise das nicht atmungsaktive Material einseitig und/oder beidseitig zumindest teilweise mit Stoff (11, 12) beschichtet belegt, kaschiert oder überzogen ist.

4. Schlankheitsgurt nach einem oder mehreren der vorhergehenden Ansprüche, **dadurch gekennzeichnet, daß** das Material (30) des hinteren Teiles (3) des Schlankheitsgurtes (2) zumindest teilweise elastisch ist.

5. Schlankheitsgurt nach einem oder mehreren der vorhergehenden Ansprüche, **dadurch gekennzeichnet, daß** im Bereich des hinteren Teiles (3) Gummistabilisatoren (31, 32) vorgesehen sind.

6. Schlankheitsgurt nach einem oder mehreren der vorhergehenden Ansprüche, **dadurch gekennzeichnet, daß** ein stabilisierender Randsaum (33, 34) oben und/oder unten im Bereich des hinteren Teiles (3) und/oder des vorderen Teiles (1) vorgesehen ist.

7. Schlankheitsgurt nach einem oder mehreren der vorhergehenden Ansprüche, **dadurch gekennzeichnet, daß** der Gurt (2) als Endlosteil ausgebildet ist.

8. Schlankheitsgurt nach Anspruch 1 - 6, **dadurch gekennzeichnet, daß** der Gurt (2) am vorderen (1) oder hinteren Teil (3) einen Verschluß (4), vorteilhafterweise einen Klettverschluß oder Knöpfe, Druckknöpfe, Haken, Bänder usw., aufweist.

9. Schlankheitsgurt nach einem oder mehreren der vorhergehenden Ansprüche, **dadurch gekennzeichnet, daß** der hintere Teil (3) 20 % bis 50 %, bevorzugt 25 % des gesamten Umfanges (20) des Schlankheitsgurtes (2) ausmacht.

10. Schlankheitsgurt nach einem oder mehreren der vorhergehenden Ansprüche, **dadurch gekennzeichnet, daß** zumindest das Material des vorderen Teiles zumindest auf der dem Körper zugewandten Seite mit einem textilartigem Material beschichtet ist.

11. Schlankheitsgurt nach einem oder mehreren der vorhergehenden Ansprüche, **dadurch gekennzeichnet, daß** das textilartige Material des vorderen Teiles im Verhältnis zur bedeckten Fläche dünn ist.

## Claims

1. Slimming belt comprising a front part and a back part, the front part being suited to be arranged in the stomach region, **characterised in that** the front part (1) of the slimming belt (2) consists of a non-breathable material (10), the front part increasing the blood circulation in the stomach region, because of the restricted air circulation of the non-breathable material (10), and thus serving for purification, and the back part (3) being at least partly breathable.

2. Slimming belt according to claim 1, **characterised in that** neoprene or another non-breathable material or a mixture of these materials is provided as material (10) of the front part (1).

3. Slimming belt according to one or both of the preceding claims, **characterised in that** the neoprene, respectively the non-breathable material, is one-sided and/or face and back at least partly coated, covered, lined or laminated with fabric (11, 12).

4. Slimming belt according to one or more of the preceding claims, **characterised in that** the material (30) of the back part (3) of the slimming belt (2) is at least partly elastic.

5. Slimming belt according to one or more of the preceding claims, **characterised in that** in the region of the back part (3) rubber stabilizers (31, 32) are provided.

6. Slimming belt according to one or more of the preceding claims, **characterised in that** a stabilising edge hem (33, 34) is provided at the top and/or at the bottom in the region of the back part (3) and/or of the front part (1).

7. Slimming belt according to one or more of the preceding claims, **characterised in that** the belt (2) is designed as an endless element.

8. Slimming belt according to claim 1 û 6, **characterised in that** the belt (2) has at its front (1) or back (3) part a fastener (4), advantageously a Velcro fastener or buttons, snap fasteners, hooks, ribbons etc.

9. Slimming belt according to one or more of the preceding claims, **characterised in that** the back part (3) comes to 20 % to 50 %, preferably 25 % of the complete circumference (20) of the slimming belt (2).

10. Slimming belt according to one or more of the preceding claims, **characterised in that** at least the material of the front part is coated with a textile-like material at least at the side facing the body.

11. Slimming belt according to one or more of the preceding claims, **characterised in that** the textile-like material of the front part is thin compared with the covered surface.

## Revendications

1. Ceinture amincissante comportant une partie ventrale, destinée à être portée au niveau du ventre, et une partie dorsale, **caractérisée en ce que** la partie ventrale (1) de la ceinture amincissante (2) consiste en un matériau (10) imperméable à l'air qui, par cette propriété, augmente la circulation sanguine au niveau du ventre et stimule la détoxication du sang des toxines qu'il contient tandis que la partie dorsale (3) est au moins partiellement perméable à l'air.

2. Ceinture amincissante selon la revendication 1, **caractérisée en ce que** le matériau (10) prévu pour la partie ventrale (1) est du Néoprène, un autre matériau imperméable à l'air ou un mélange de ces matériaux.

3. Ceinture amincissante selon une ou deux des revendications précédentes, **caractérisée en ce que** le Néoprène ou le matériau imperméable à l'air est recouvert, au moins partiellement, sur un côté ou des deux côtés par un tissu (11, 12) qui est déposé, collé ou tendu.

4. Ceinture amincissante selon une ou plusieurs des revendications précédentes, **caractérisée en ce que** le matériau (30) de la partie dorsale (3) de la ceinture amincissante (2) est au moins partiellement élastique.

5. Ceinture amincissante selon une ou plusieurs des revendications précédentes, **caractérisée en ce que** des stabilisateurs en caoutchouc (31, 32) sont prévus au niveau de la partie dorsale (3).

6. Ceinture amincissante selon une ou plusieurs revendications précédentes, **caractérisée en ce qu'**un ourlet stabilisant (33, 34) existe en haut et/ou en bas de la partie ventrale (1) et/ou de la partie dorsale (3).

7. Ceinture amincissante selon une ou plusieurs des revendications précédentes, **caractérisée en ce que** la ceinture (2) est fabriquée de façon à produire un tube.

8. Ceinture amincissante selon les revendications 1 à 6, **caractérisée en ce que** la ceinture (2) possède une fermeture (4) au niveau de la partie ventrale (1) ou de la partie dorsale (3) et de préférence une fermeture de type velcro ou des boutons, des boutons pression, des crochets, des lacets, etc.

9. Ceinture amincissante selon une ou plusieurs des revendications précédentes, **caractérisée en ce que** la partie dorsale (3) représente entre 20% et 50% et de préférence 25% de la circonférence totale (20) de la ceinture amincissante (2).

10. Ceinture amincissante selon une ou plusieurs des revendications précédentes, **caractérisée en ce qu'**au moins le matériau de la partie ventrale possède une couche de textile et ceci au moins du côté du corps.

11. Ceinture amincissante selon une ou plusieurs des revendications précédentes, **caractérisée en ce que** le matériau textile de la partie ventrale est fin par rapport à la surface qu'il couvre.
